# FASCICULE DE BREVET EUROPEEN

(11) **EP 0 588 672 B1**
(45) Date de publication et mention de la délivrance du brevet: **17.12.1997**
(21) Numéro de dépôt: 93401464.8
(22) Date de dépôt: 09.06.1993
(51) Int. Cl.: A61M 1/00

(54) **Valve implantable pour le traitement de l'hydrocéphalie**
Implantierbares Ventil für die Behandlung von Hydrocephalie
Implantable valve for the treatment of hydrocephalus

(30) Priorité: 15.09.1992 FR 9210972
(43) Date de publication de la demande: 23.03.1994
(73) Titulaire: CORDIS S.A., F-91170 Viry-Châtillon (FR)
(72) Inventeur: Lecuyer, Alain, F-06130 Grasse (FR)
(74) Mandataire: Geismar, Thierry

(56) Documents cités:
- FR-A- 2 372 366
- US-A- 4 551 128
- US-A- 4 676 772

## Description

La présente invention concerne une valve implantable pour le traitement de l'hydrocéphalie, et plus particulièrement une telle valve comprenant une chambre délimitée par des parois, un orifice d'entrée susceptible d'être relié à une zone à drainer et entouré par un siège pour un organe d'obturation de l'orifice d'entrée, des moyens élastiques pour presser l'organe d'obturation contre son siège, et un orifice de sortie susceptible d'être relié à une zone de drainage, ledit siège étant monté sur une paroi formée d'une membrane.

De telles valves, connues par exemple par le document FR-A-2 372 366, sont utilisées pour contrôler le drainage d'un excès de fluide céphalo-rachidien contenu dans les ventricules cérébraux vers, par exemple, la cavité péritonéale. Elles s'ouvrent au-delà d'un certain seuil de pression différentielle entre leur orifice d'entrée et leur orifice de sortie, et empêchent ensuite cette pression différentielle de dépasser la valeur de seuil.

Ces valves présentent toutefois le sérieux inconvénient de ne pas tenir compte des différences de hauteur de colonne de liquide en fonction de la position, notamment debout ou couché, du patient. En position debout, il se produit en effet dans la chambre une dépression due à la différence de niveau entre la zone d'implantation de la valve et la zone de drainage.

La présente invention vise à pallier ces inconvénients en fournissant une valve dont la pression différentielle d'ouverture correspond effectivement à la différence de pression entre la pression régnant dans la zone à drainer et la pression ambiante.

A cet effet, la présente invention a pour objet une valve du type ci-dessus, caractérisée par le fait que la surface extérieure de la membrane est soumise à la pression environnante.

Ainsi, lorsqu'il se produit dans la chambre une dépression artificielle, due par exemple à la position debout du patient, la membrane se déforme avec pour effet un déplacement du siège et par conséquent une déformation des moyens élastiques. Il en résulte qu'est alors nécessaire, pour permettre un décollement de l'organe d'obturation de son siège, une plus grande différence de pression entre l'orifice d'entrée dans la valve et la chambre, ce qui compense la dépression régnant dans la chambre.

L'organe d'obturation peut être constitué par une bille appliquée sur un siège conique, par exemple par un ressort de compression.

Avantageusement, la valve selon ce mode de réalisation comporte un capot de protection de la membrane, ledit capot présentant des trous pour la mise à la pression environnante de la surface extérieure de la membrane.

On décrira maintenant, à titre d'exemple non limitatif, deux modes de réalisation particuliers de l'invention, en référence aux dessins annexés, dans lesquels :
- la figure 1 est une vue en coupe axiale d'une valve selon l'invention, et
- les figures 2a et 2b illustrent le fonctionnement de cette valve.

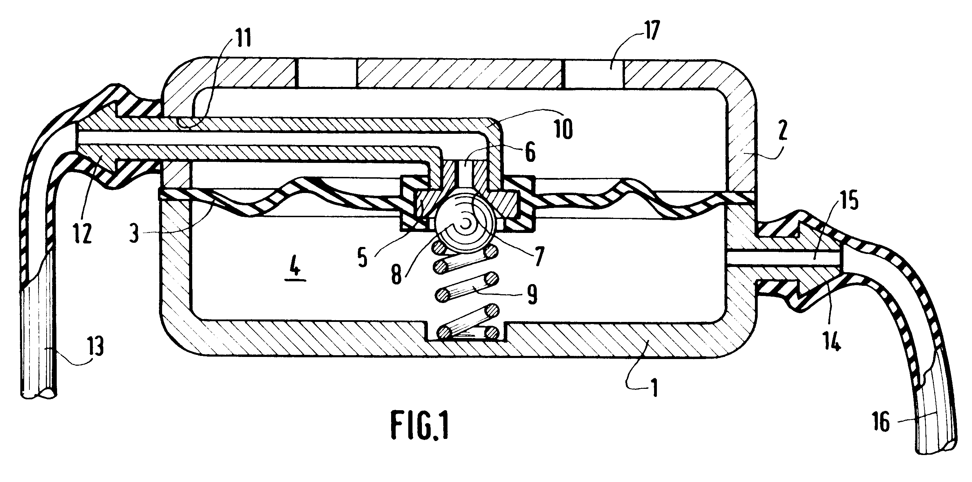

La valve de la figure 1 est contenue dans un boîtier composé d'un demi boîtier de fond 1 et d'un demi boîtier supérieur de protection 2. Une membrane 3 disposée entre les deux demi boîtiers délimite avec le demi boîtier de fond 1 une chambre 4.

La membrane 3 comporte dans sa partie contrale un orifice dans lequel est maintenu par tout moyen convenable une pièce 5 perçée en son centre d'un orifice 6 et formant du côté de la chambre 4 un siège conique 7.

Une bille 8 est disposée dans la chambre 4 et est pressée sur le siège 7 par un ressort de compression 9 prenant appui, d'une part sur la bille 8 et, d'autre part, sur la paroi de fond du demi boîtier 1.

Un conduit souple 10 est emmanché sur la pièce 5 autour de l'orifice 6, traverse l'espace délimité par la membrane 3 et le demi boîtier 2, sort du demi boîtier 2 par un trou 11, et forme à l'extérieur de la valve un raccord 12 sur lequel est emmanché un cathéter 13.

Le demi boîtier 1 forme par ailleurs un raccord 14 muni d'un orifice 15, par lequel la chambre 4 communique avec un cathéter 16 emmanché sur le raccord 14.

Enfin, le demi boîtier 2 comporte des trous 17 par lesquels la surface de la membrane extérieure à la chambre 4 est mise à la pression environnante.

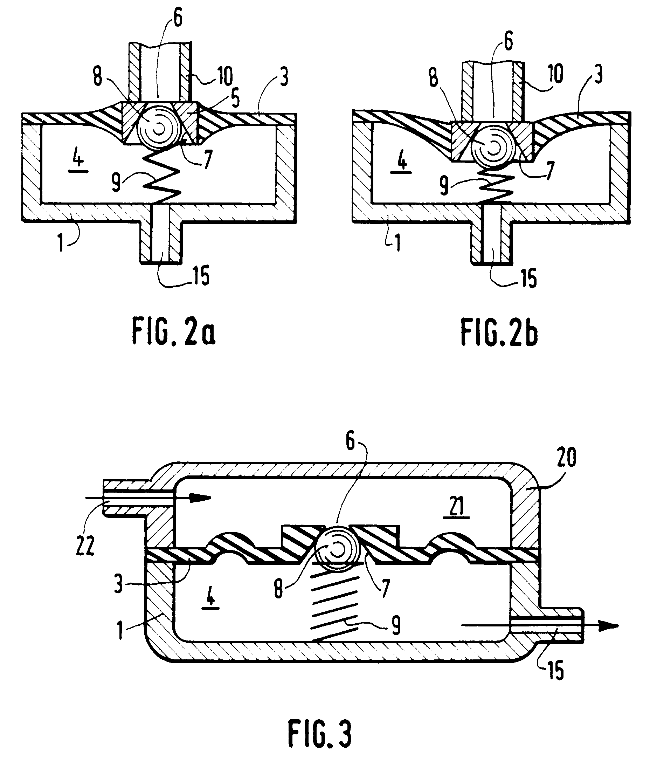

La valve qui vient d'être décrite fonctionne comme représentée schématiquement aux figures 2a et 2b.

La figure 2a représente la valve dans sa configuration "normale", par exemple lorsque le patient sur lequel elle est implantée est en position couchée. Dans cette position, la pression dans la chambre 4 est égale à la pression environnante, de sorte que la membrane 3 est dans son état non contraint. Le ressort 9 possède une élongation donnée et applique la bille 8 sur le siège 7 avec une force telle que la valve s'ouvre pour une pression différentielle donnée entre son orifice d'entrée 6 et la chambre 4, c'est-à-dire pratiquement entre la zone à drainer et la pression environnante.

S'il se produit une dépression dans la chambre 4, par exemple à la suite du redressement du patient, la membrane 3 se déforme vers l'intérieur de la chambre 4, comme représenté à la figure 2b. Le ressort 9 est par conséquent comprimé, de sorte que la pression différentielle entre l'orifice 6 et la chambre 4 nécessaire à l'ouverture de la valve augmente. Mais la pression dans la chambre 4 est maintenant inférieure à la pression environnante, de sorte que la pression différentielle entre l'orifice 6 et la pression environnante est la même que dans le cas de la figure 2a si les caractéristiques de la membrane 3 et du ressort 9 ont été choisies convenablement, ce qui est à la portée de l'homme de métier.

La valve selon l'invention permet par conséquent de imiter les surdrainages dus aux pressions hydrostatiques engendrées par les changements de position du patient.

## Revendications

1. Valve implantable pour le traitement de l'hydrocéphalie, comprenant une chambre (4) délimitée par des parois (1,3), un orifice d'entrée (6) susceptible d'être relié à une zone à drainer et entouré par un siège (7) pour un organe d'obturation (8) de l'orifice d'entrée, des moyens élastiques (9) pour presser l'organe d'obturation contre son siège, et un orifice de sortie (15) susceptible d'être relié à une zone de drainage, ledit siège étant monté sur une paroi formée d'une membrane (3), caractérisée par le fait que la surface extérieure de la membrane est soumise à la pression environnante.

2. Valve selon la revendication 1, dans laquelle l'organe d'obturation est une bille et le siège est conique.

3. Valve selon l'une quelconque des revendications 1 et 2, dans lequel les moyens élastiques sont un ressort de compression.

4. Valve selon l'une quelconque des revendications 1 à 3, comportant un capot de protection (2) de la membrane, ledit capot présentant des trous (17) pour la mise à la pression environnante de la surface extérieure de la membrane.

## Claims

1. Implantable valve for the treatment of hydrocephalus, comprising a chamber (4) marked out by walls (1, 3), an inlet orifice (6) which is likely to be linked to zone to be drained and surrounded by seating (7) for a sealing organ (8) for the inlet orifice, elastic means (9) to press the sealing organ against its seating, and an outlet orifice (15) which is likely to be linked to a draining zone, the said seating being assembled on a wall formed from a membrane (3) characterised by the face that the outer surface of the membrane is subject to the environmental pressure.

2. Valve according to claim 1, in which the sealing organ is a ball and the seating is conical.

3. Valve according to any of claims 1 and 2, in which the elastic means is a compression spring.

4. Valve according to any of claims 1 to 3, comprising a protection cap (2) for the membrane, the said cap having holes (17) to put the outer surface of the membrane under environmental pressure.

## Patentansprüche

1. Implantierbares Ventil zur Behandlung von Hydrocephalus mit einer Kammer (4), welche von Wänden (1, 3) begrenzt wird, einer Eintrittsöffnung (6), welche mit einer zur Drainage bestimmten Zone verbindbar ist und von einem Sitz (7) für ein Verschlußorgan (8) für die Eintrittsöffnung umgeben ist, mit elastischen Mitteln (9) zum Andrücken des Verschlußorgans gegen den Sitz, und einer Austrittsöffnung (15), welche mit einer Drainagezone verbindbar ist, wobei der Sitz auf einer von einer Membrane (3) gebildeten Wand vorgesehen ist, **dadurch gekennzeichnet**, daß die Außenfläche der Membrane dem Umgebungsdruck ausgesetzt ist.

2. Ventil nach Anspruch 1, bei dem das Verschlußorgan eine Kugel ist, und der Sitz konisch ausgebildet ist.

3. Ventil nach Anspruch 1 oder 2, bei dem die elastischen Mittel von einer Kompressionsfeder gebildet werden.

4. Ventil nach einem der Ansprüche 1 bis 3, welches eine Schutzabdeckung (2) für die Membrane aufweist, wobei die Abdeckung Öffnungen (17) besitzt, um den Umgebungsdruck der Außenfläche auf die Membrane zur Einwirkung zu bringen.
